Europäisches Patentamt

European Patent Office

Office européen des brevets

(19)

(11) Numéro de publication : **0 395 532 B1**

(12) **FASCICULE DE BREVET EUROPEEN**

(45) Date de publication du fascicule du brevet :
**29.06.94 Bulletin 94/26**

(51) Int. Cl.⁵ : **C12Q 1/04, C12Q 1/34,**
**// C12R1:42**

(21) Numéro de dépôt : **90401158.2**

(22) Date de dépôt : **27.04.90**

(54) **Perfectionnement d'un milieu d'isolement pour l'identification de la bactérie salmonella.**

(30) Priorité : **27.04.89 FR 8905594**
**06.07.89 FR 8909114**

(43) Date de publication de la demande :
**31.10.90 Bulletin 90/44**

(45) Mention de la délivrance du brevet :
**29.06.94 Bulletin 94/26**

(84) Etats contractants désignés :
**AT BE CH DE DK ES FR GB GR IT LI LU NL SE**

(56) Documents cités :
**EP-A- 0 163 867**
**EP-A- 0 206 277**
**GB-A- 2 050 418**
**APPLIED & ENVIRONMENTAL MICROBIO-**
**LOGY, vol. 34, no. 5, novembre 1977, American**
**Society for Microbiology, US; J.Y. D'AOUST,**
**pp. 595-596**

(56) Documents cités :
**BIOCHEMICAL JOURNAL, vol. 231, 1985, GB;**
**J. ROS et al., pp. 145-149**
**BIOLOGICAL ABSTRACTS, vol. 80, no. 8, 1985,**
**Philadelphia, PA (US); Y. ICHIKAWA et al., p.**
**774, no. 71128**
**BIOLOGICAL ABSTRACTS, vol. 82, no. 9, 1986,**
**Philadelphia, PA (US); M. VERZELE et al., p.**
**AB-71, no. 79489**

(73) Titulaire : **Rambach, Alain**
**73 Bld du Montparnasse**
**F-75006 Paris (FR)**

(72) Inventeur : **Rambach, Alain**
**73 Bld du Montparnasse**
**F-75006 Paris (FR)**

(74) Mandataire : **Warcoin, Jacques et al**
**Cabinet Régimbeau**
**26, avenue Kléber**
**F-75116 Paris (FR)**

EP 0 395 532 B1

## Description

La présente invention se rapporte à un milieu d'isolement permettant l'identification des bactéries du genre Salmonella.

L'identification de la bactérie Salmonella pathogène pour l'homme est un problème majeur de la bactériologie médicale et de la surveillance de l'hygiène agro-alimentaire.

Ainsi, dans le cas d'épidémies transmises par des élevages de poules, les volailles infestées au niveau du tractus intestinal ne sont pas malades mais constituent un réservoir de Salmonella. Celles-ci peuvent être propagées, notamment par les oeufs, dans l'alimentation à la suite de ces épidémies. D'ailleurs, la Salmonella est une bactérie à déclaration obligatoire.

Aujourd'hui, il devient nécessaire de prévoir la mise en oeuvre à grande échelle de la détection des sites infectés et plus particulièrement des fermes infectées par la bactérie Salmonella pour réduire ces épidémies.

En fait, les Salmonella sont généralement à reconnaître parmi les espèces commensales, Escherichia Coli et Proteus.

La détection de Salmonella est habituellement réalisée sur un milieu gélosé d'isolement sélectif pour entérobactéries permettant la différenciation des entérobactéries pathogènes et la détection des colonies suspectes Salmonella. Un milieu d'isolement idéal doit permettre la croissance d'entérobactéries, la différenciation des diverses espèces présentes afin de permettre l'identification ultérieure d'une colonie de chaque type, et la détection des colonies suspectes Salmonella.

Or, les milieux d'isolement d'entérobactéries de l'art antérieur, ne répondent que partiellement à la détection de Salmonella. En effet, on rencontre des bactéries commensales fréquentes, de type Proteus mirabilis qui se révèlent des faux positifs sur ces milieux.

Parmi les milieux sélectifs gélosés proposés, le milieu Hektoen est généralement préféré, tout en étant l'un des plus chers.

Ainsi, sur ce milieu Hektoen, les bactéries Salmonella donnent des colonies bleues à centre noir (lac⁻sac⁻sal⁻H₂S⁺), et la bactérie mirabilis qui a les mêmes propriétés donne également des colonies bleues à centre noir. Ces faux positifs obligent donc à un surcroît de travail et de dépenses car ils rendent obligatoire l'examen ultérieur de plusieurs colonies suspectes au lieu d'une, éventuellement sans résultat positif, sans parler du risque d'erreur de diagnostic après une lecture rapide.

De plus, l'ambiguïté de la réponse Salmonella requiert aussi pour le passage à l'étape ultérieure de réisolement de colonies et de différenciation biochimique, une plus grande qualification que pour une simple reconnaissance d'une coloration de colonies. La différenciation biochimique peut enfin retarder l'identification et donc imposer un délai supplémentaire.

Les milieux d'isolement de l'état de la technique sont notamment cités dans GB-A-2 050 418 et EP-A-0 206 277.

L'objet de la présente invention est précisément de remédier à ces inconvénients et de proposer un milieu d'isolement permettant la détection de colonies de Salmonella, de manière non ambigüe par une coloration spécifique de la colonie. La présente invention n'utilise en effet pas les caractères classiques H₂S⁺lac⁻sac⁻sal⁻ de repérages de Salmonella qui laissent apparaître comme faux positifs les Proteus.

Plus précisément, la présente invention se rapporte à un milieu d'isolement pour l'identification de la bactérie Salmonella caractérisé en ce qu'à un support de culture contenant des peptones, on ajoute un diol contenant 2 à 10 atomes de carbone métabolisables par Salmonella et un indicateur de pH réagissant à l'acidification.

Selon un mode de réalisation particulier de l'invention, ce diol métabolisable par Salmonella, est adsorbé sur un matériau pulvérulent.

Le matériau utilisé a une faible granulométrie, c'est-à-dire de préférence inférieure à environ 100 μm de façon à permettre une adsorption efficace dudit diol et à assurer une bonne fluidité à la poudre ainsi obtenue.

Ainsi, on utilise à titre de matériau pulvérulent de préférence de la silice fine, et plus particulièrement du gel de silice. Toutefois, il peut également être fait usage d'autres types de matériaux tels la cellulose.

Parmi les diols utilisables, il faut citer plus particulièrement l'éthanediol, les propanediols et les butanediols, notamment le 1,2 propanediol. Ces diols sont en effet métabolisés par la bactérie Salmonella pour donner des espèces acides capables de faire réagir des indicateurs de pH tels que le rouge neutre. Au contraire, les espèces commensales telles que E. coli et Proteus ne donnent pas ce type de réaction.

Ce milieu est plus particulièrement utilisable pour la détection de Salmonella alimentaires qui donnent toutes des résultats positifs sur ce milieu. Pour la mise en oeuvre du procédé, on préfère utiliser des milieux de culture gélosés appropriés pour la culture en colonies qui permet d'identifier plus rapidement les colonies de Salmonella.

Parmi les milieux favorisant le développement des entérobactéries par exemple, on pourra utiliser un milieu contenant du désoxycholate. Si l'on souhaite augmenter la précision de la détection, on peut ajouter au milieu en cause un substrat de la béta-galactosidase notamment un substrat chromogène de celle-ci type bromo chloro indoxyl galactoside, bromo naphtyl galactoside ou hydroxyquinoline galactoside et, éventuellement en présence d'un inducteur tel que l'IPTG. En effet la souche de Salmonella étant béta gal⁻, elle ne réagit pas sur ce substrat.

L'intérêt des diol selon l'invention, lorsque l'on utilise les deux caractères d'identification précédents, est que la présence de polyols ne masque pas le caractère béta gal⁺ à la différence de la plupart des hydrates de carbone qui, par répression catabolique, peuvent masquer le caractère béta gal⁺. Lorsque ces deux caractères d'identification sont visualisés par des réactions colorées, on peut identifier par un choix judicieux les différents types de bactéries particulièrement Salmonella, Proteus et E. coli. Ainsi, le rouge neutre comme indicateur de pH et l'X-gal comme substrat de la béta-galactosidase permettent de distinguer Salmonella de E. coli, Citrobacter et Proteus dans un mélange les contenant comme cela apparaîtra à la lecture des exemples ci-dessous.

Il est bien entendu possible dans ce type de milieu d'utiliser d'autres caractères négatifs de Salmonella par exemple béta glu⁻, dans ce cas, on utilisera un substrat de la béta-glucosidase.

De même, il est possible plutôt que de mettre en oeuvre un milieu gélosé, notamment des plaques, d'utiliser d'autres supports tel que support papier, colonne et même de détecter dans le milieu lui-même.

La présente invention concerne également un procédé permettant l'identification de Salmonella dans lequel on cultive l'échantillon sur un milieu selon l'invention et on détecte la présence de Salmonella par la réaction de l'indicateur de pH à l'acidification du milieu.

Les exemples donnés ci-dessous à titre non limitatif permettront de mettre en évidence d'autres avantages et caractéristiques de la présente invention.

**EXEMPLE 1 :**

**Milieu d'isolement permettant la détection des Salmonella**

On réalise un milieu ayant la composition suivante :

| Constituants | g/litre d'eau |
|---|---|
| . 1,2 propane-diol | 5 |
| . Peptones | 5 |
| . Extrait de levure | 2 |
| . Désoxycholate | 1 |
| . Bromo Chloro Indoxyl Galactoside | 0,1 |
| . Rouge neutre | 0,03 |
| . Agar | 15 |

Par ensemencement de ce milieu avec différents types d'entérobactéries et après 48 heures de culture, on obtient les résultats suivants : les Salmonella donnent des colonies rouges, les E. coli des colonies bleu-vert, les Citrobacter des colonies bleu-violet et les Proteus des colonies incolores.

**EXEMPLE 2 :**

**Milieu d'isolement selon l'invention, le polyol étant adsorbé sur un matériau pulvérulent.**

Le milieu utilisé a la composition suivante :

| Constituants | g/litre d'eau |
|---|---|
| - 1,2 propanediol/gel de silice (10g/16g) | 26 |
| - Peptones | 5 |
| - Extrait de levure | 2 |
| - Désoxycholate | 1 |
| - Bromo Chloro Indoxyl Galactoside | 0,1 |
| - Rouge neutre | 0,03 |
| - Agar | 15 |

Par ensemencement de ce milieu avec différents types d'entérobactéries et après 48 heures de culture, on obtient des résultats analogues à ceux observés avec le milieu de culture objet de l'exemple 1, à savoir : les Salmonella donnent des colonies rouges, les E. Coli des colonies bleu-vert, les Citrobacter des colonies bleu-violet, et les Proteus des colonies incolores.

**Revendications**

1. Milieu d'isolement pour l'identification des bactéries du genre Salmonella, caractérisé en ce qu'à un support de culture contenant des peptones, on ajoute un diol contenant 2 à 10 atomes de carbone métabolisables par Salmonella et un indicateur de pH réagissant à l'acidification.

2. Milieu d'isolement selon la revendication 1, caractérisé en ce que le diol métabolisable par Salmonella est adsorbé sur un matériau pulvérulent

3. Milieu d'isolement selon la revendication 2, caractérisé en ce que le matériau pulvérulent présente une granulométrie inférieure à environ 100 µm.

4. Milieu d'isolement selon la revendication 2 ou 3, caractérisé en ce que le matériau pulvérulent est de préférence choisi parmi la silice fine, le gel de silice et la cellulose.

5. Milieu d'isolement selon l'une des revendications 1 à 4, caractérisé en ce que le diol est choisi de préférence parmi l'éthanediol, les butanediols et les propanediols.

6. Milieu d'isolement selon l'une des revendications 1 à 5 caractérisé en ce que le diol choisi est le propanediol 1,2.

7. Milieu d'isolement selon l'une des revendications 1 à 6, caractérisé en ce que l'indicateur de pH est le rouge neutre.

8. Milieu d'isolement selon l'une des revendications 1 à 7, caractérisé en ce que le support de culture est un milieu gélosé approprié pour la culture en colonies.

9. Milieu d'isolement selon l'une des revendications 1 à 8, caractérisé en ce que le milieu contient en outre des désoxycholates.

10. Milieu d'isolement selon l'une des revendications 1 à 9, caractérisé en ce que le milieu contient également un substrat chromogène de la béta galactosidase.

11. Milieu d'isolement selon l'une des revendications 1 à 10, caractérisé en ce qu'il contient également de l'isopropyl-béta-D-thiogalactopyranoside (IPTG).

12. Procédé d'identification de la présence de bactéries Salmonella dans un échantillon, caractérisé en ce qu'on cultive l'échantillon sur un milieu selon l'une des revendications 1 à11, et en ce que l'on détermine la présence de Salmonella par la réaction de l'indicateur de pH à l'acidification du milieu.

4

EP 0 395 532 B1

## Patentansprüche

1.	Isoliermedium zur Isolierung von Bakterien der Art <u>Salmonella</u>, dadurch gekennzeichnet, daß zu einem Kulturträger, der Peptone enthält, ein Diol mit 2 bis 10 Kohlenstoffatomen, metabolisierbar durch <u>Salmonella</u> und ein pH-Indikator, der auf Ansäuern reagiert, hinzugefügt wird.

2.	Isoliermedium nach Anspruch 1, dadurch gekennzeichnet, daß das von <u>Salmonella</u> metabolisierbare Diol auf einem Pulvermaterial adsorbiert ist.

3.	Isoliermedium nach Anspruch 2, dadurch gekennzeichnet, daß das Pulvermaterial eine Kornabstufung unter etwa 100 μm aufweist.

4.	Isoliermedium nach Anspruch 2 oder 3, **dadurch gekennzeichnet, daß** das das Pulvermaterial bevorzugt aus feiner Kieselerde, Silicagel und Cellulose ausgewählt ist.

5.	Isoliermedium nach einem der Ansprüche 1 bis 4, dadurch gekennzeichnet, daß das Diol bevorzugt aus Ethandiol, den Butandiolen und den Propandiolen ausgewählt ist.

6.	Isoliermedium nach einem der Ansprüche 1 bis 5, dadurch gekennzeichnet, daß das ausgewählte Diol 1,2-Propandiol ist.

7.	Isoliermedium nach einem der Ansprüche 1 bis 6, dadurch gekennzeichnet, daß der Indikator Neutralrot ist.

8.	Isoliermedium nach einem der Ansprüche 1 bis 7, dadurch gekennzeichnet, daß der Kulturträger ein Agar-Agar-Medium für die Kultur in Kolonien ist.

9.	Isoliermedium nach einem der Ansprüche 1 bis 8, dadurch gekennzeichnet, daß das Medium darüberhinaus Desoxycholate enthält.

10.	Isoliermedium nach einem der Ansprüche 1 bis 9, dadurch gekennzeichnet, daß das Medium darüberhinaus ein chromogenes Substrat aus beta-Galactosidase enthält.

11.	Isoliermedium nach einem der Ansprüche 1 bis 10, dadurch gekennzeichnet, daß es darüberhinaus Isopropyl-beta-D-thiogalactopyranosid (IPTG) enthält.

12.	Verfahren zur Identifizierung der Gegenwart von <u>Salmonella</u>-Bakterien in einer Probe, dadurch gekennzeichnet, daß man die Probe auf einem Medium nach einem der Ansprüche 1 bis 11 kultiviert, und daß man die Gegenwart von <u>Salmonella</u> durch Reaktion des pH-Indikators durch Ansäuren des Mediums bestimmt.

## Claims

1.	An isolating medium for the identification of the <u>Salmonella</u> bacterium, characterized in that a diol containing 2 to 10 carbon atoms metabolizable by <u>Salmonella</u> and a pH indicator reacting to acidification are added to a culture support containing peptones.

2.	The isolating medium as claimed in claim 1, wherein the diol metabolizable by <u>Salmonella</u> is adsorbed on a pulverulent material

3.	The isolating medium as claimed in claim 2, characterized in that the pulverulent material has a particle size of less than about 100 μm.

4.	The isolating medium as claimed in claim 2 or 3, wherein the pulverulent material is preferably chosen from fine silica, silica gel and cellulose.

5.	The isolating medium as claimed in one of claims 1 to 4, wherein the diol is preferably chosen from ethanediol.

5

6. The isolating medium as claimed in one of claims 1 to 5 wherein the diol chosen is 1,2-propanediol.

7. The isolating medium as claimed in one of claims 1 to 6 wherein the pH indicator is neutral red.

8. The isolating medium as claimed in one of claims 1 to 7 wherein the culture support is an agar medium appropriate for the culture of colonies.

9. The isolating medium as claimed in one of claims 1 to 8 wherein the medium also contains deoxycholates.

10. The isolating medium as claimed in one of claims 1 to 9 wherein the medium also contains a chromogenic beta-galactosidase substrate.

11. The isolating medium as claimed in one of claims 1 to 10 which also contains isopropyl-beta-D-thiogalactopyranoside (IPTG).

12. A process for identifying the presence of <u>Salmonella</u> bacteria in a sample, which comprises culturing the sample on a medium as claimed in one of claims 1 to 11 and determining the presence of <u>Salmonella</u> by the reaction of the pH indicator to the acidification of the medium.